# EUROPEAN PATENT APPLICATION

(11) **EP 4 501 337 A1**
(43) Date of publication of application: **05.02.2025**
(21) Application number: 23780949.6
(22) Date of filing: 30.03.2023
(51) Int. Cl.: A61K 35/30, A61P 27/02, C12N 5/071

(54) **CORNEAL ENDOTHELIAL CELL PREPARATION CONTAINING NO CULTURE-DERIVED COMPONENT, AND METHOD FOR PRODUCING SAME**

(30) Priority: 31.03.2022 JP 2022060148
(71) Applicant: The Doshisha, Kyoto 602-8580 (JP); ActualEyes Inc., Kyotanabe-shi, Kyoto 610-0332 (JP)
(72) Inventor: KOIZUMI, Noriko, Kyotanabe-shi, Kyoto 610-0394 (JP); OKUMURA, Naoki, Kyotanabe-shi, Kyoto 610-0394 (JP); MATSUOKA, Yasushi, Kyotanabe-shi, Kyoto 610-0332 (JP)
(74) Representative: Vossius & Partner Patentanwälte Rechtsanwälte mbB
(86) International application number: PCT/JP2023/013321
(87) International publication number: WO 2023/190941

(57) **Abstract**

The present disclosure provides: a method for washing corneal endothelial cells; and a method for producing a corneal endothelial cell preparation. In one aspect, the present disclosure provides a method for producing a corneal endothelial cell preparation, the method comprising a step for washing a corneal endothelial cell or a corneal endothelium-like cell with a solution containing about 0.01% by weight to about 10% by weight of a protein and a step for mixing the corneal endothelial cell or the corneal endothelium-like cell with a composition for preparation production use to produce a corneal endothelial preparation, in which the protein is selected from the group consisting of human serum albumin, casein, lactoferrin, ovalbumin, and combinations thereof.

## Description

### [Technical Field]

The present disclosure relates to a method for cleaning corneal endothelial cells and a method for producing a corneal endothelial cell formulation, and a corneal endothelial cell formulation produced by such a method.

### [Background Art]

When corneal endothelial cells are damaged, the cornea becomes cloudy, resulting in severe visual impairment due to bullous keratopathy. Although the only treatment for bullous keratopathy is corneal transplantation, there are problems such as a shortage of donors and rejection. Therefore, development of a new regenerative medicine treatment is desired. The present inventors have established a treatment method for corneal endothelial regeneration by proliferating corneal endothelial cells isolated from the cornea of a donor with the addition of a ROCK inhibitor and injecting the cells into many patients. On the other hand, it is necessary to recover cells from an incubator and remove one such as fetal bovine serum which has been added upon culture in order to safely administer the cells to patients. However, with the conventional method, only about 50% of cells could be recovered due to cell damage during cleaning or adhesion to a container.

### [Summary of Invention]

### [Solution to Problem]

The present inventors have examined in detail a method for adding a stabilizing component during cell cleaning and found a method for efficiently cleaning cells without decreasing a cell recovery rate by adding an appropriate amount of a clinically usable protein component such as human serum albumin.

The present disclosure provides, for example, the following items.
(Item 1) A method for producing a corneal endothelial cell formulation, the method including:
   cleaning corneal endothelial cells or corneal endothelium-like cells with a solution including about 0.01% by weight to about 10% by weight of a protein; and
   mixing the corneal endothelial cells or the corneal endothelial-like cells with a formulating composition to produce a corneal endothelial cell formulation,
   the protein being selected from the group consisting of human serum albumin, casein, lactoferrin, ovalbumin, and a combination thereof.
(Item 2) The method according to the above item, in which the cleaning includes suspending the corneal endothelial cells or the corneal endothelium-like cells in the solution to provide a suspension of the corneal endothelial cells or the corneal endothelium-like cells and centrifuging the suspension to recover the corneal endothelial cells or the corneal endothelium-like cells.
(Item 3) The method according to any one of the above items, in which the cleaning removes an impurity in the corneal endothelial cell formulation.
(Item 4) The method according to any one of the above items, in which a loss rate of the corneal endothelial cells or the corneal endothelial-like cells in the cleaning is 20% or less.
(Item 5) The method according to any one of the above items, in which a loss rate of the corneal endothelial cells or the corneal endothelial-like cells in the cleaning is 15% or less.
(Item 6) The method according to any one of the above items, in which a loss rate of the corneal endothelial cells or the corneal endothelial-like cells in the cleaning is 10% or less.
(Item 7) The method according to any one of the above items, in which the protein is human serum albumin.
(Item 8) The method according to any one of the above items, in which the solution includes about 0.5% by weight to about 10% by weight of human serum albumin.
(Item 9) The method according to any one of the above items, in which the solution includes about 1% by weight to about 5% by weight of human serum albumin.
(Item 10) The method according to any one of the above items, in which the solution includes about 1% by weight to about 3% by weight of human serum albumin.
(Item 11) The method according to any one of the above items, in which the solution includes about 2% by weight of human serum albumin.
(Item 12) The method according to any one of the above items, in which the solution is a culture medium, a buffer solution, or an intraocular perfusate, or includes one or more component(s) of the culture medium, the buffer solution, or the intraocular perfusate.
(Item 13) The method according to any one of the above items, in which the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution, or OPEGUARD.
(Item 14) The method according to any one of the above items, in which the component of the culture medium includes calcium, magnesium, potassium, sodium, a phosphate, a bicarbonate, a vitamin, an essential amino acid, or a combination thereof.
(Item 15) The method according to any one of the above items, in which the component of the culture medium further includes transferrin, insulin, hypoxanthine, thymidine, sodium bicarbonate, sodium pyruvate, L-glutamic acid, or a combination thereof.
(Item 16) The method according to any one of the above items, in which the formulating composition is a cell preservation solution, a culture medium, a buffer solution, or an intraocular perfusate, or includes one or more component(s) of the cell preservation solution, the culture medium, the buffer solution, or the intraocular perfusate.
(Item 17) The method according to any one of the above items, in which the cell preservation solution is CryoStor (registered trademark) CS2, CryoStor (registered trademark) CS5, Bambanker (registered trademark), or CellBanker (registered trademark).
(Item 18) The method according to any one of the above items, in which the component of the cell preservation solution includes DMSO, glycerin, polyethylene glycol, or a combination thereof.
(Item 19) A method for cleaning corneal endothelial cells or corneal endothelium-like cells, the method including:
   suspending the corneal endothelial cells or the corneal endothelium-like cells in a solution including about 0.01% by weight to about 10% by weight of a protein to provide a suspension of the corneal endothelial cells or the corneal endothelium-like cells; and
   centrifuging the suspension to recover the corneal endothelial cells or the corneal endothelium-like cells,
   the protein being selected from the group consisting of human serum albumin, casein, lactoferrin, ovalbumin, and a combination thereof.
(Item 19A) The method according to any one of the above items, in which the cleaning removes an impurity in the corneal endothelial cell formulation.
(Item 19B) The method according to any one of the above items, in which a loss rate of the corneal endothelial cells or the corneal endothelial-like cells in the cleaning is 20% or less.
(Item 19C) The method according to any one of the above items, in which a loss rate of the corneal endothelial cells or the corneal endothelial-like cells in the cleaning is 15% or less.
(Item 19D) The method according to any one of the above items, in which a loss rate of the corneal endothelial cells or the corneal endothelial-like cells in the cleaning is 10% or less.
(Item 19E) The method according to any one of the above items, in which the protein is human serum albumin.
(Item 19F) The method according to any one of the above items, in which the solution includes about 0.5% by weight to about 10% by weight of human serum albumin.
(Item 19G) The method according to any one of the above items, in which the solution includes about 1% by weight to about 5% by weight of human serum albumin.
(Item 19H) The method according to any one of the above items, in which the solution includes about 1% by weight to about 3% by weight of human serum albumin.
(Item 19I) The method according to any one of the above items, in which the solution includes about 2% by weight of human serum albumin.
(Item 19J) The method according to any one of the above items, in which the solution is a culture medium, a buffer solution, or an intraocular perfusate, or includes one or more component(s) of the culture medium, the buffer solution, or the intraocular perfusate.
(Item 19K) The method according to any one of the above items, in which the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution, or OPEGUARD.
(Item 19L) The method according to any one of the above items, in which the component of the culture medium includes calcium, magnesium, potassium, sodium, a phosphate, a bicarbonate, a vitamin, an essential amino acid, or a combination thereof.
(Item 20) A cell cleaning solution for corneal endothelial cells or corneal endothelium-like cells, the solution including
   about 0.01% by weight to about 10% by weight of a protein selected from the group consisting of human serum albumin, casein, lactoferrin, ovalbumin, and a combination thereof.
(Item 21) The cell cleaning solution according to any one of the above items, in which cleaning of the corneal endothelial cells is performed by suspending the corneal endothelial cells or the corneal endothelium-like cells in the cell cleaning solution to provide a suspension of the corneal endothelial cells or the corneal endothelium-like cells and centrifuging the suspension to recover the corneal endothelial cells or the corneal endothelium-like cells.
(Item 22) The cell cleaning solution according to any one of the above items, in which the cleaning removes an impurity in the corneal endothelial cell formulation.
(Item 23) The cell cleaning solution according to any one of the above items, in which a loss rate of the corneal endothelial cells or the corneal endothelial-like cells in the cleaning is 20% or less.
(Item 24) The cell cleaning solution according to any one of the above items, in which a loss rate of the corneal endothelial cells or the corneal endothelial-like cells in the cleaning is 15% or less.
(Item 25) The cell cleaning solution according to any one of the above items, in which a loss rate of the corneal endothelial cells or the corneal endothelial-like cells in the cleaning is 10% or less.
(Item 26) The cell cleaning solution according to any one of the above items, in which the protein is human serum albumin.
(Item 27) The cell cleaning solution according to any one of the above items, in which the solution includes about 0.5% by weight to about 10% by weight of human serum albumin.
(Item 28) The cell cleaning solution according to any one of the above items, in which the solution includes about 1% by weight to about 5% by weight of human serum albumin.
(Item 29) The cell cleaning solution according to any one of the above items, in which the solution includes about 1% by weight to about 3% by weight of human serum albumin.
(Item 30) The cell cleaning solution according to any one of the above items, in which the solution includes about 2% by weight of human serum albumin.
(Item 31) The cell cleaning solution according to any one of the above items, in which the solution is a culture medium or a buffer solution, or includes one or more component(s) of the culture medium or the buffer solution.
(Item 32) The cell cleaning solution according to any one of the above items, in which the culture medium is Opti-MEM (registered trademark), DMEM, MEM, RPMI 1640, Ham's F-12, PBS, HEPES, Hanks' Balanced Salt Solution (HBSS), a saline solution, an oxyglutathione solution.
(Item 33) The cell cleaning solution according to any one of the above items, in which the component of the culture medium includes calcium, magnesium, potassium, sodium, a phosphate, a bicarbonate, a vitamin, an essential amino acid, or a combination thereof.
(Item 34) The cell cleaning solution according to any one of the above items, in which the component of the culture medium further includes transferrin, insulin, hypoxanthine, thymidine, sodium bicarbonate, sodium pyruvate, L-glutamic acid, or a combination thereof.
(Item 35) A composition for producing a corneal endothelial cell formulation in a suspension state, the composition including
   about 0.01% by weight to about 10% by weight of a protein selected from the group consisting of human serum albumin, casein, lactoferrin, ovalbumin, and a combination thereof.
(Item 35A) The composition according to any one of the above items, in which the composition is brought into contact with the corneal endothelial cells or the corneal endothelium-like cells that have been cultured, the corneal endothelial cells are subjected to cleaning, and the cleaning of the corneal endothelial cells is performed by suspending the corneal endothelial cells or the corneal endothelium-like cells in the composition to provide a suspension of the corneal endothelial cells or the corneal endothelium-like cells and centrifuging the suspension to recover the corneal endothelial cells or the corneal endothelium-like cells.
(Item 35B) The composition according to any one of the above items, in which the cleaning removes an impurity in the corneal endothelial cell formulation.
(Item 35C) The composition according to any one of the above items, in which a loss rate of the corneal endothelial cells or the corneal endothelial-like cells in the cleaning is 20% or less.
(Item 35D) The composition according to any one of the above items, in which a loss rate of the corneal endothelial cells or the corneal endothelial-like cells in the cleaning is 15% or less.
(Item 35E) The composition according to any one of the above items, in which a loss rate of the corneal endothelial cells or the corneal endothelial-like cells in the cleaning is 10% or less.
(Item 35F) The composition according to any one of the above items, in which the protein is human serum albumin.
(Item 35G) The composition according to any one of the above items, in which the composition includes about 0.5% by weight to about 10% by weight of human serum albumin.
(Item 35H) The composition according to any one of the above items, in which the composition includes about 1% by weight to about 5% by weight of human serum albumin.
(Item 35I) The composition according to any one of the above items, in which the composition includes about 1% by weight to about 3% by weight of human serum albumin.
(Item 35J) The composition according to any one of the above items, in which the composition includes about 2% by weight of human serum albumin.
(Item 35K) The composition according to any one of the above items, in which the composition is a culture medium, a buffer solution, or an intraocular perfusate, or includes one or more component(s) of the culture medium, the buffer solution, or the intraocular perfusate.
(Item 35L) The composition according to any one of the above items, in which the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution.
(Item 35M) The composition according to any one of the above items, in which the component of the culture medium includes calcium, magnesium, potassium, sodium, a phosphate, a bicarbonate, a vitamin, an essential amino acid, or a combination thereof.
(Item 35N) The composition according to any one of the above items, in which the component of the culture medium further includes transferrin, insulin, hypoxanthine, thymidine, sodium bicarbonate, sodium pyruvate, L-glutamic acid, or a combination thereof.
(Item A1) Use of a protein in cell cleaning of corneal endothelial cells or corneal endothelium-like cells, the protein being used in a cell cleaning solution including about 0.01% by weight to about 10% by weight the protein, and the protein being selected from the group consisting of human serum albumin, casein, lactoferrin, ovalbumin, and a combination thereof.
(Item A2) The use according to any one of the above items, in which cleaning of the corneal endothelial cells is performed by suspending the corneal endothelial cells or the corneal endothelium-like cells in the cell cleaning solution to provide a suspension of the corneal endothelial cells or the corneal endothelium-like cells and centrifuging the suspension to recover the corneal endothelial cells or the corneal endothelium-like cells.
(Item A3) The use according to any one of the above items, in which the cleaning removes an impurity in the corneal endothelial cell formulation.
(Item A4) The use according to any one of the above items, in which a loss rate of the corneal endothelial cells or the corneal endothelial-like cells in the cleaning is 20% or less.
(Item A5) The use according to any one of the above items, in which a loss rate of the corneal endothelial cells or the corneal endothelial-like cells in the cleaning is 15% or less.
(Item A6) The use according to any one of the above items, in which a loss rate of the corneal endothelial cells or the corneal endothelial-like cells in the cleaning is 10% or less.
(Item A7) The use according to any one of the above items, in which the protein is human serum albumin.
(Item A8) The use according to any one of the above items, in which the cell cleaning solution includes about 0.5% by weight to about 10% by weight of human serum albumin.
(Item A9) The use according to any one of the above items, in which the cell cleaning solution includes about 1% by weight to about 5% by weight of human serum albumin.
(Item A10) The use according to any one of the above items, in which the cell cleaning solution includes about 1% by weight to about 3% by weight of human serum albumin.
(Item A11) The use according to any one of the above items, in which the cell cleaning solution includes about 2% by weight of human serum albumin.
(Item A12) The use according to any one of the above items, in which the solution is a culture medium or a buffer solution, or includes one or more component(s) of the culture medium or the buffer solution.
(Item A13) The use according to any one of the above items, in which the culture medium is Opti-MEM (registered trademark), DMEM, MEM, RPMI 1640, Ham's F-12, PBS, HEPES, Hanks' Balanced Salt Solution (HBSS), a saline solution, an oxyglutathione solution.
(Item A14) The use according to any one of the above items, in which the component of the culture medium includes calcium, magnesium, potassium, sodium, a phosphate, a bicarbonate, a vitamin, an essential amino acid, or a combination thereof.
(Item A15) The use according to any one of the above items, in which the component of the culture medium further includes transferrin, insulin, hypoxanthine, thymidine, sodium bicarbonate, sodium pyruvate, L-glutamic acid, or a combination thereof.
(Item A16) Use of a protein in producing a corneal endothelial cell formulation in a suspension state, the protein being used in a composition including about 0.01% by weight to about 10% by weight the protein, and the protein being selected from the group consisting of human serum albumin, casein, lactoferrin, ovalbumin, and a combination thereof.
(Item A17) The use according to any one of the above items, in which the composition is brought into contact with the corneal endothelial cells or the corneal endothelium-like cells that have been cultured, the corneal endothelial cells are subjected to cleaning, and the cleaning of the corneal endothelial cells is performed by suspending the corneal endothelial cells or the corneal endothelium-like cells in the composition to provide a suspension of the corneal endothelial cells or the corneal endothelium-like cells and centrifuging the suspension to recover the corneal endothelial cells or the corneal endothelium-like cells.
(Item A18) The use according to any one of the above items, in which the cleaning removes an impurity in the corneal endothelial cell formulation.
(Item A19) The use according to any one of the above items, in which a loss rate of the corneal endothelial cells or the corneal endothelial-like cells in the cleaning is 20% or less.
(Item A20) The use according to any one of the above items, in which a loss rate of the corneal endothelial cells or the corneal endothelial-like cells in the cleaning is 15% or less.
(Item A21) The use according to any one of the above items, in which a loss rate of the corneal endothelial cells or the corneal endothelial-like cells in the cleaning is 10% or less.
(Item A22) The use according to any one of the above items, in which the protein is human serum albumin.
(Item A23) The use according to any one of the above items, in which the composition includes about 0.5% by weight to about 10% by weight of human serum albumin.
(Item A24) The use according to any one of the above items, in which the composition includes about 1% by weight to about 5% by weight of human serum albumin.
(Item A25) The use according to any one of the above items, in which the composition includes about 1% by weight to about 3% by weight of human serum albumin.
(Item A26) The use according to any one of the above items, in which the composition includes about 2% by weight of human serum albumin.
(Item A27) The use according to any one of the above items, in which the composition is a culture medium, a buffer solution, or an intraocular perfusate, or includes one or more component(s) of the culture medium, the buffer solution, or the intraocular perfusate.
(Item A28) The use according to any one of the above items, in which the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution.
(Item A29) The use according to any one of the above items, in which the component of the culture medium includes calcium, magnesium, potassium, sodium, a phosphate, a bicarbonate, a vitamin, an essential amino acid, or a combination thereof.
(Item A30) The use according to any one of the above items, in which the component of the culture medium further includes transferrin, insulin, hypoxanthine, thymidine, sodium bicarbonate, sodium pyruvate, L-glutamic acid, or a combination thereof.
(Item B1) A protein for cell cleaning of corneal endothelial cells or corneal endothelium-like cells, the protein being used in a cell cleaning solution including about 0.01% by weight to about 10% by weight the protein, and the protein being selected from the group consisting of human serum albumin, casein, lactoferrin, ovalbumin, and a combination thereof.
(Item B2) The protein according to any one of the above items, in which cleaning of the corneal endothelial cells is performed by suspending the corneal endothelial cells or the corneal endothelium-like cells in the cell cleaning solution to provide a suspension of the corneal endothelial cells or the corneal endothelium-like cells and centrifuging the suspension to recover the corneal endothelial cells or the corneal endothelium-like cells.
(Item B3) The protein according to any one of the above items, in which the cleaning removes an impurity in the corneal endothelial cell formulation.
(Item B4) The protein according to any one of the above items, in which a loss rate of the corneal endothelial cells or the corneal endothelial-like cells in the cleaning is 20% or less.
(Item B5) The protein according to any one of the above items, in which a loss rate of the corneal endothelial cells or the corneal endothelial-like cells in the cleaning is 15% or less.
(Item B6) The protein according to any one of the above items, in which a loss rate of the corneal endothelial cells or the corneal endothelial-like cells in the cleaning is 10% or less.
(Item B7) The protein according to any one of the above items, in which the protein is human serum albumin.
(Item B8) The protein according to any one of the above items, in which the cell cleaning solution includes about 0.5% by weight to about 10% by weight of human serum albumin.
(Item B9) The protein according to any one of the above items, in which the cell cleaning solution includes about 1% by weight to about 5% by weight of human serum albumin.
(Item B10) The protein according to any one of the above items, in which the cell cleaning solution includes about 1% by weight to about 3% by weight of human serum albumin.
(Item B11) The protein according to any one of the above items, in which the cell cleaning solution includes about 2% by weight of human serum albumin.
(Item B12) The protein according to any one of the above items, in which the solution is a culture medium or a buffer solution, or includes one or more component(s) of the culture medium or the buffer solution.
(Item B13) The protein according to any one of the above items, in which the culture medium is Opti-MEM (registered trademark), DMEM, MEM, RPMI 1640, Ham's F-12, PBS, HEPES, Hanks' Balanced Salt Solution (HBSS), a saline solution, an oxyglutathione solution.
(Item B14) The protein according to any one of the above items, in which the component of the culture medium includes calcium, magnesium, potassium, sodium, a phosphate, a bicarbonate, a vitamin, an essential amino acid, or a combination thereof.
(Item B15) The protein according to any one of the above items, in which the component of the culture medium further includes transferrin, insulin, hypoxanthine, thymidine, sodium bicarbonate, sodium pyruvate, L-glutamic acid, or a combination thereof.
(Item B16) A protein for producing a corneal endothelial cell formulation in a suspension state, the protein being used in a composition including about 0.01% by weight to about 10% by weight the protein, and the protein being selected from the group consisting of human serum albumin, casein, lactoferrin, ovalbumin, and a combination thereof.
(Item B17) The protein according to any one of the above items, in which the composition is brought into contact with the corneal endothelial cells or the corneal endothelium-like cells that have been cultured, the corneal endothelial cells are subjected to cleaning, and the cleaning of the corneal endothelial cells is performed by suspending the corneal endothelial cells or the corneal endothelium-like cells in the composition to provide a suspension of the corneal endothelial cells or the corneal endothelium-like cells and centrifuging the suspension to recover the corneal endothelial cells or the corneal endothelium-like cells.
(Item B18) The protein according to any one of the above items, in which the cleaning removes an impurity in the corneal endothelial cell formulation.
(Item B19) The protein according to any one of the above items, in which a loss rate of the corneal endothelial cells or the corneal endothelial-like cells in the cleaning is 20% or less.
(Item B20) The protein according to any one of the above items, in which a loss rate of the corneal endothelial cells or the corneal endothelial-like cells in the cleaning is 15% or less.
(Item B21) The protein according to any one of the above items, in which a loss rate of the corneal endothelial cells or the corneal endothelial-like cells in the cleaning is 10% or less.
(Item B22) The protein according to any one of the above items, in which the protein is human serum albumin.
(Item B23) The protein according to any one of the above items, in which the composition includes about 0.5% by weight to about 10% by weight of human serum albumin.
(Item B24) The protein according to any one of the above items, in which the composition includes about 1% by weight to about 5% by weight of human serum albumin.
(Item B25) The protein according to any one of the above items, in which the composition includes about 1% by weight to about 3% by weight of human serum albumin.
(Item B26) The protein according to any one of the above items, in which the composition includes about 2% by weight of human serum albumin.
(Item B27) The protein according to any one of the above items, in which the composition is a culture medium, a buffer solution, or an intraocular perfusate, or includes one or more component(s) of the culture medium, the buffer solution, or the intraocular perfusate.
(Item B28) The protein according to any one of the above items, in which the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution.
(Item B29) The protein according to any one of the above items, in which the component of the culture medium includes calcium, magnesium, potassium, sodium, a phosphate, a bicarbonate, a vitamin, an essential amino acid, or a combination thereof.
(Item B30) The protein according to any one of the above items, in which the component of the culture medium further includes transferrin, insulin, hypoxanthine, thymidine, sodium bicarbonate, sodium pyruvate, L-glutamic acid, or a combination thereof.
(Item C1) A corneal endothelial cell formulation, the formulation being produced by
   cleaning corneal endothelial cells or corneal endothelium-like cells with a solution including about 0.01% by weight to about 10% by weight of a protein; and
   mixing the corneal endothelial cells or the corneal endothelial-like cells with a formulating composition to produce a corneal endothelial cell formulation,
   the protein being selected from the group consisting of human serum albumin, casein, lactoferrin, ovalbumin, and a combination thereof.
(Item C2) The corneal endothelial cell formulation according to the above item, in which the cleaning includes suspending the corneal endothelial cells or the corneal endothelium-like cells in the solution to provide a suspension of the corneal endothelial cells or the corneal endothelium-like cells and centrifuging the suspension to recover the corneal endothelial cells or the corneal endothelium-like cells.
(Item C3) The corneal endothelial cell formulation according to any one of the above items, in which the cleaning removes an impurity in the corneal endothelial cell formulation.
(Item C4) The corneal endothelial cell formulation according to any one of the above items, in which a loss rate of the corneal endothelial cells or the corneal endothelial-like cells in the cleaning is 20% or less.
(Item C5) The corneal endothelial cell formulation according to any one of the above items, in which a loss rate of the corneal endothelial cells or the corneal endothelial-like cells in the cleaning is 15% or less.
(Item C6) The corneal endothelial cell formulation according to any one of the above items, in which a loss rate of the corneal endothelial cells or the corneal endothelial-like cells in the cleaning is 10% or less.
(Item C7) The corneal endothelial cell formulation according to any one of the above items, in which the protein is human serum albumin.
(Item C8) The corneal endothelial cell formulation according to any one of the above items, in which the solution includes about 0.5% by weight to about 10% by weight of human serum albumin.
(Item C9) The corneal endothelial cell formulation according to any one of the above items, in which the solution includes about 1% by weight to about 5% by weight of human serum albumin.
(Item C10) The corneal endothelial cell formulation according to any one of the above items, in which the solution includes about 1% by weight to about 3% by weight of human serum albumin.
(Item C11) The corneal endothelial cell formulation according to any one of the above items, in which the solution includes about 2% by weight of human serum albumin.
(Item C12) The corneal endothelial cell formulation according to any one of the above items, in which the solution is a culture medium, a buffer solution, or an intraocular perfusate, or includes one or more component(s) of the culture medium, the buffer solution, or the intraocular perfusate.
(Item C13) The corneal endothelial cell formulation according to any one of the above items, in which the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution, or OPEGUARD.
(Item C14) The corneal endothelial cell formulation according to any one of the above items, in which the component of the culture medium includes calcium, magnesium, potassium, sodium, a phosphate, a bicarbonate, a vitamin, an essential amino acid, or a combination thereof.
(Item C15) The corneal endothelial cell formulation according to any one of the above items, in which the component of the culture medium further includes transferrin, insulin, hypoxanthine, thymidine, sodium bicarbonate, sodium pyruvate, L-glutamic acid, or a combination thereof.
(Item C16) The corneal endothelial cell formulation according to any one of the above items, in which the formulating composition is a cell preservation solution, a culture medium, a buffer solution, or an intraocular perfusate, or includes one or more component(s) of the cell preservation solution, the culture medium, the buffer solution, or the intraocular perfusate.
(Item C17) The corneal endothelial cell formulation according to any one of the above items, in which the cell preservation solution is CryoStor (registered trademark) CS2, CryoStor (registered trademark) CS5, Bambanker (registered trademark), or CellBanker (registered trademark).
(Item C18) The corneal endothelial cell formulation according to any one of the above items, in which the component of the cell preservation solution includes DMSO, glycerin, polyethylene glycol, or a combination thereof.

In the present invention, it is intended that one or more of the above features may be provided in additional combinations in addition to the explicit combinations. Those skilled in the art will appreciate further embodiments and advantages of the present invention upon reading and understanding the following detailed description, if necessary.

### [Advantageous Effects of Invention]

The present disclosure provides a cell formulation from which a component derived from a different origin such as fetal bovine serum has been removed by cleaning with a protein that can be administered to the human body upon preparation of the cell formulation in regenerative medicine. The present invention can reduce loss upon cell recovery in the process of cell cleaning by adding a protein that can be administered to the human body to a cell cleaning solution.

### [Brief Description of Drawings]

[FIG. 1] Figure 1 shows an overview of Examples 1 to 4;
[FIG. 2] Figure 2 shows a graph of a loss rate due to addition of casein to a cell cleaning solution using an immortalized corneal endothelial cell line (iHCEC). Error bars indicate mean ± SD. Statistical significance is based on Dunnett's t-test (vs 0%) (n = 3, **p < 0.01, *p < 0.05);
[FIG. 3] Figure 3 shows a graph of a loss rate due to addition of lactoferrin to a cell cleaning solution using an immortalized corneal endothelial cell line (iHCEC). Error bars indicate mean ± SD. Statistical significance is based on Dunnett' s t-test (vs 0%) (n = 3, **p < 0.01, *p < 0.05);
[FIG. 4] Figure 4 shows a graph of a loss rate due to addition of ovalbumin to a cell cleaning solution using an immortalized corneal endothelial cell line (iHCEC). Error bars indicate mean ± SD. Statistical significance is based on Dunnett' s t-test (vs 0%) (n = 3, **p < 0.01, *p < 0.05);
[FIG. 5] Figure 5 shows a graph of a loss rate due to addition of human serum albumin to a cell cleaning solution using an immortalized corneal endothelial cell line (iHCEC). Error bars indicate mean ± SD. Statistical significance is based on Dunnett' s t-test (vs 0%) (n = 3, **p < 0.01, *p < 0.05);
[FIG. 6] Figure 6 shows an overview of Example 5;
[FIG. 7] Figure 7 shows a graph of a loss rate due to addition of human serum albumin to a cell cleaning solution using human corneal endothelial cells after 4 passages. Error bars indicate mean ± SD. Statistical significance is based on Dunnett' s t-test (vs 0%) (n = 3, **p < 0.01); and
[FIG. 8] Figure 8 shows representative photographs of results of Example 7.

### [Description of Embodiments]

The present invention will be described. It should be understood that singular expressions also include the concept of their plural forms throughout the specification, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art, unless otherwise noted. Therefore, unless otherwise defined, all technical and scientific terms used herein have the same meaning as generally understood by those skilled in the art to which the present invention pertains. In the event of a conflict, this specification (including definitions) shall prevail. As used herein, the term "about" means ± 10% of the value that follows.

### (Definitions)

As used herein, the phrase "corneal endothelial cells" is used in the usual sense used in the art. The cornea is one of lamellar tissues that make up the eye, is transparent, and is located closest to the outside world. In humans, the cornea is composed of five layers: the corneal epithelium, the Bowman's membrane, the lamina propria, the Descemet's membrane (basement membrane of corneal endothelium), and the corneal endothelium from the outside (body surface). Unless otherwise specified, portions of the cornea other than the epithelium and the endothelium are sometimes collectively referred to as the "corneal stroma" and are so referred to herein. As used herein, the term "HCEC" is an abbreviation for human corneal endothelial cells.

As used herein, the phrase "corneal endothelium-like cells" refers to cells differentiated from stem cells, such as iPS cells, that have substantially the same functions as corneal endothelial cells. Methods of differentiating stem cells, such as embryonic stem cells (ES cells) and induced pluripotent stem cells (iPS cells), into corneal endothelium-like cells are well known in the art (McCabe et al., PLoS One. 2015 Dec 21; 10 (12): e0145266; Ali et al., Invest Ophthalmol Vis Sci. 2018 May 1; 59 (6): 2437-2444). In a typical example, briefly, iPS cells are seeded in 35 mm Matrigel-coated plates (Corning) at a 1:12 dilution on day 0 using a cell dissociation buffer (Life Technologies) (a plate with 80% confluent is divided into 12 plates). The iPS cells are grown in a medium (mTeSR1; STEMCELL Technologies Inc.) for 4 days. On day 4, the mTeSR1 medium was replaced with a Smad inhibitor medium containing 500 ng/mL human recombinant Noggin (R&D Systems, Minneapolis, MN, USA) and 10 µM SB431542 (Millipore Sigma) in a basal medium composed of 80% DMEM-F12 (Life Technologies), 20% KSR (Life Technologies), 1% non-essential amino acids (Life Technologies), 1 mM L-glutamine (STEMCELL Technologies, Inc.), 0.1 mM β-mercaptoethanol (Millipore Sigma), and 8 ng/mL βFGF (Millipore Sigma). On day 6, the Smad inhibitor medium was replaced with a corneal medium containing 0.1 x B27 supplement (Life Technologies), 10 ng/mL recombinant human platelet-derived growth factor-BB (PDGF-BB; PeproTech, Rocky Hill, NJ, USA) and 10 ng/mL recombinant human Dickkopf-related protein-2 (DKK-2; R&D Systems) in a basal medium composed of 80% DMEM-F12 (Life Technologies), 20% KSR (Life Technologies), 1% non-essential amino acids (Life Technologies), 1 mM L-glutamine (STEMCELL Technologies, Inc.), 0.1 mM β-mercaptoethanol (Millipore Sigma), and 8 ng/mL βFGF (Millipore Sigma). On day 7, differentiating CECs are transferred to a new Matrigel-coated plate (35 mm) and grown in the corneal medium for an additional 13 days. Differentiated CECs are collected on day 20. The above-mentioned example is a typical example, and those skilled in the art may use other methods well known in the art (Fukuta et al., PLoS One. 2014 Dec 2; 9 (12): e112291; Hayashi et al., Nature. 2016 Mar 17; 531 (7594): 376-80). In addition, those skilled in the art can produce corneal endothelium-like cells by adjusting conditions of methods well known in the art as appropriate.

The phrases "corneal endothelial cells" and "corneal endothelium-like cells" may contain a magnetic substance (e.g., iron). For example, when corneal endothelial cells containing a magnetic material are injected into the anterior chamber, magnetic force can encourage the cells to be attracted and adhered to the inner cornea (e.g., Descemet's membrane) (Patel et al., Invest Ophthalmol Vis Sci. 2009 May; 50(5): 2123-31; Mimura et al., Exp Eye Res. 2003 Jun; 76(6): 745-51; and Mimura et al., Exp Eye Res. 2005 Feb; 80(2): 149-57). The phrase "magnetic substance" refers to a substance that is magnetized by a magnetic field, such as iron, cobalt, nickel, and ferrite.

As used herein, the phrase "corneal endothelial cell formulation" refers to a formulation that includes corneal endothelial cells or corneal endothelium-like cells and that is in a form suitable for use or a form for prior preparation. The phrase "prior preparation" refers to preparation of a formulation suitable for use by adding an agent thereto or diluting it with a solvent immediately prior to administration.

As used herein, the term "cleaning" refers to removal of an impurity in a corneal endothelial cell formulation. The phrase "impurity in a corneal endothelial cell formulation" refers to a component derived from a different origin to be added during cell culture (e.g., fetal bovine serum), a component undesirable for human administration such as an antibiotic that may be added to a culture medium (e.g., gentamicin), an enzyme inhibitor (e.g., a p38 MAPK inhibitor such as SB203580), a waste product and cytokines (e.g., IL-6, IL-8) that cells release into a medium.

As used herein, the phrase "loss rate" refers to a percentage of cells lost after cleaning with a cell cleaning solution according to the present disclosure. It is calculated as a percentage of the number of cells after cleaning, taking the number of cells before cleaning as 100%.

### (Preferred Embodiment)

Although preferred embodiments will be described below, it should be understood that these embodiments are illustrative of the present invention and the scope of the present invention is not limited to such preferred embodiments. It should also be understood that those skilled in the art can easily make modifications, changes, etc. within the scope of the present invention with reference to the following preferred examples. Those skilled in the art may combine any of these embodiments as appropriate.

### (Methods for producing corneal endothelial cell formulation and cleaning corneal endothelial cells)

In one aspect, the present disclosure provides a method for producing a corneal endothelial cell formulation, the method including cleaning corneal endothelial cells or corneal endothelium-like cells with a solution including a protein that may be administered to a human; and mixing the corneal endothelial cells or the corneal endothelium-like cells with a formulating composition to produce a corneal endothelial cell formulation. In some embodiments, the protein that may be administered to a human can be selected from the group consisting of human serum albumin, casein, lactoferrin, ovalbumin, and a combination thereof. In some embodiments, the protein that may be administered to a human can be included in the solution at about 0.01% by weight to about 10% by weight.

In one aspect, the present disclosure provides a method for producing a corneal endothelial cell formulation, the method including cleaning corneal endothelial cells or corneal endothelium-like cells with a solution including about 0.01% by weight to about 10% by weight of a protein; and mixing the corneal endothelial cells or the corneal endothelium-like cells with a formulating composition to produce a corneal endothelial cell formulation, the protein being selected from the group consisting of human serum albumin, casein, lactoferrin, ovalbumin, and a combination thereof.

In one aspect, the present disclosure provides a method for cleaning corneal endothelial cells or corneal endothelium-like cells, the method including suspending corneal endothelial cells or corneal endothelium-like cells in a solution including a protein that may be administered to a humans to provide a suspension of the corneal endothelial cells or the corneal endothelium-like cells; and centrifuging the suspension to recover the corneal endothelial cells or corneal endothelium-like cells. In some embodiments, the protein that may be administered to a human can be selected from the group consisting of human serum albumin, casein, lactoferrin, ovalbumin, and a combination thereof. In some embodiments, the protein that may be administered to a human can be included in the solution at about 0.01% by weight to about 10% by weight.

In one aspect, the present disclosure provides a method for cleaning corneal endothelial cells or corneal endothelium-like cells, the method including suspending corneal endothelial cells or corneal endothelium-like cells in a solution including about 0.01% by weight to about 10% by weight of a protein to provide a suspension of the corneal endothelial cells or the corneal endothelium-like cells; and centrifuging the suspension to recover the corneal endothelial cells or corneal endothelium-like cells, the protein being selected from the group consisting of human serum albumin, casein, lactoferrin, ovalbumin, and a combination thereof.

In some embodiments, the cleaning may be performed once or multiple times, preferably twice. In order to reduce loss due to the cleaning, the cleaning is preferably performed up to five times.

In some embodiments, a cell concentration when cells are suspended in a cell cleaning solution can be at least about 0.5 × 10⁴ cells/mL, at least about 1.0 × 10⁴ cells/mL, at least about 0.5 × 10⁵ cells/mL, at least about 1.0 × 10⁶ cells/mL, at least about 0.5 x 10⁷ cells/mL, or at least about 1.0 × 10⁷ cells/mL. The cell concentration is preferably about 1.0 × 10⁶ cells/mL to about 1.0 × 10⁷ cells/mL and can be up to about 0.5 × 10⁸ cells/mL.

Cells according to the present disclosure may be used without reculturing after cleaning, since the loss of the cells in cleaning is low and an impurity has been extremely reduced therefrom. This is particularly advantageous in clinical use. In some embodiments, the cells according to the present disclosure may be cleaned and then suspended into a suspension appropriate for the intended use without reculturing. In some embodiments, the cells according to the present disclosure are cells for clinical use (e.g., for cell infusion therapy) and can be suspended and administered without reculturing after cleaning.

In some embodiments, the cleaning may include suspending the corneal endothelial cells or the corneal endothelium-like cells in the solution to provide a suspension of the corneal endothelial cells or the corneal endothelium-like cells and centrifuging the suspension to recover the corneal endothelial cells or the corneal endothelium-like cells. The cleaning can remove an impurity in the corneal endothelial cell formulation. Examples of the impurity in the corneal endothelial cell formulation include, but not limited to, any component derived from a different origin (e.g., fetal bovine serum), any antibiotic (e.g., gentamicin), any enzyme inhibitor (e.g., a p38 MAPK inhibitor such as SB203580), any waste product or any cytokine (e.g., IL-6, IL-8) that cells release into a medium. In some embodiments, the cleaning can remove at least one of fetal bovine serum, gentamicin, a p38MAPK inhibitor, IL-6, and IL-8.

The method according to the present disclosure can reduce a loss rate of the corneal endothelial cells or corneal endothelial-like cells in the cleaning. In some embodiments, the loss rate of the corneal endothelial cells or the corneal endothelium-like cells in the cleaning can be 20% or less, preferably 15% or less, and more preferably 10% or less.

In some embodiments, a protein included in a solution for cleaning can be human serum albumin. In a certain embodiment, an amount of the human serum albumin included in the solution for cleaning can be about 0.5% by weight to about 10% by weight, preferably about 1% by weight to about 5% by weight, more preferably about 1% by weight to about 3% by weight, and most preferably about 2% by weight.

In a certain embodiment, the protein included in a solution for cleaning can be casein. In a certain embodiment, an amount of the casein included in the solution for cleaning can be from about 0.01% by weight to about 1% by weight, preferably from about 0.03% by weight to about 0.3% by weight, and more preferably about 0.1% by weight.

In a certain embodiment, the protein included in a solution for cleaning can be lactoferrin. In a certain embodiment, an amount of the lactoferrin included in the solution for cleaning can be about 0.5% by weight to about 5% by weight, preferably about 1% by weight to about 4% by weight, more preferably about 2% by weight to about 3% by weight, and most preferably about 3% by weight.

In some embodiments, the protein included in a solution for cleaning can be ovalbumin. In a certain embodiment, an amount of the ovalbumin included in the solution for cleaning can be from about 0.01% by weight to about 1% by weight, preferably from about 0.03% by weight to about 0.5% by weight, more preferably from about 0.03% by weight to about 0.3% by weight, further preferably from about 0.03% by weight to about 0.1% by weight, and most preferably about 0.03% by weight.

In some embodiments, a solution to be used in the method according to the present disclosure may be a culture medium or a buffer solution, or may include one or more component(s) of the culture medium or the buffer solution. Examples of the culture medium, the buffer solution, or an intraocular perfusate include, but are not limited to, Opti-MEM (registered trademark), a Dulbecco's Modified Eagle's Medium (DMEM), a Minimum Essential Medium (MEM), RPMI 1640, Ham's F-12, PBS HEPES, Hanks' Balanced Salt Solution (HBSS), a saline solution, and an oxyglutathione solution. In a certain embodiment, the component of the culture medium may include calcium, magnesium, potassium, sodium, a phosphate, a bicarbonate, a vitamin, an essential amino acid, or a combination thereof. In a further embodiment, the component of the culture medium may include transferrin, insulin, hypoxanthine, thymidine, sodium bicarbonate, sodium pyruvate, L-glutamic acid, or a combination thereof, in addition to the culture medium or component(s) of the culture medium.

Examples of the Buffer solution include, but not limited to, an acetate buffer solution, a phosphate buffer solution, a citrate buffer solution, a HEPES buffer solution, a Tris buffer solution, an ammonium chloride buffer solution, and a phosphate buffered saline solution. In some embodiments, a pH can be adjusted to between about 6.0 and about 8.0 and preferably between about 6.2 and about 7.7. In a preferred embodiment, the buffer solution can be a phosphate buffer solution.

Examples of the intraocular perfusate include, but not limited to, oxyglutathione or OPEGUARD.

In some embodiments, a formulating composition may be a cell preservation solution, a culture medium, a buffer solution, or an intraocular perfusate, or may include one or more component(s) of the cell preservation solution, the culture medium, the buffer solution, or the intraocular perfusate. Examples of the cell preservation solution include, but not limited to, CryoStor (registered trademark) CS2, CryoStor (registered trademark) CS5, Bambanker (registered trademark), and CellBanker (registered trademark). In a certain embodiment, dimethyl sulfoxide (DMSO), glycerin, polyethylene glycol, or a combination thereof may be included. In some embodiments, DMSO, glycerin, polyethylene glycol, or a combination thereof may be added to the above-mentioned culture medium to produce a cell preservation solution.

In a certain embodiment, an amount of DMSO included in the cell preservation solution can be about 1 to about 10% by weight, preferably about 1 to about 5% by weight, more preferably about 1 to about 3% by weight, and most preferably about 2% by weight.

In a certain embodiment, an amount of glycerin included in the cell preservation solution can be from about 1 to about 20% by weight, preferably from about 5 to about 15% by weight, more preferably from about 7 to about 13% by weight, and most preferably about 10% by weight.

In a certain embodiment, an amount of polyethylene glycol included in the cell preservation solution can be from about 1 to about 20% by weight, preferably from about 5 to about 15% by weight, more preferably from about 7 to about 13% by weight, and most preferably about 10% by weight.

### (Corneal endothelial cell cleaning solution and composition for producing formulation)

In one aspect, the present disclosure provides a cell cleaning solution for corneal endothelial cells or corneal endothelium-like cells including about 0.01% by weight to about 10% by weight of a protein, the protein being selected from the group consisting of human serum albumin, casein, lactoferrin, ovalbumin, and a combination thereof.

In another aspect, the present disclosure provides a composition for producing a corneal endothelial cell formulation in a suspension state, the composition including about 0.01% by weight to about 10% by weight of a protein, the protein being selected from the group consisting of human serum albumin, casein, lactoferrin, ovalbumin, and a combination thereof.

In some embodiments, cleaning of corneal endothelial cells can be performed by suspending the corneal endothelial cells or the corneal endothelium-like cells in a cell cleaning solution to provide a suspension of the corneal endothelial cells or the corneal endothelium-like cells and centrifuging the suspension to recover the corneal endothelial cells or the corneal endothelium-like cells. This cleaning can remove an impurity in the corneal endothelial cell formulation.

Embodiments of a loss rate of the corneal endothelial cells or the corneal endothelium-like cells, an amount of the protein, the culture medium, and the buffer solution are as described above.

The present invention has been described above with preferred embodiments for ease of understanding. Hereinafter, the present invention will be described with reference to Examples, but the above description and the following examples are provided for illustrative purposes only and are not intended to limit the present invention. Accordingly, the scope of the present invention is not limited to embodiments or examples specifically described herein, but is limited only by the claims.

### [Example]

The present disclosure will be specifically described with reference to Examples. It is understood that the various reagents used in Examples can be obtained from Sigma-Aldrich, BASF Japan K.K., and the like, in addition to those specifically indicated.

### (Example 1: Reduction of loss rate by adding casein to cell cleaning solution)

### (Preparation example: Production immortalized corneal endothelial cell line (iHCEC))

In this example, an immortalized corneal endothelial cell line (iHCEC) was produced from a research donor cornea derived from a healthy donor.

### (Culture method)

Corneal endothelial cells and a basement membrane were mechanically detached from a research cornea purchased from Seattle Eye Bank, and the basement membrane was lysed with a collagenase to collect corneal endothelial cells, which were then primary-cultured. For a medium, a 3T3 feeder cell-conditioned medium which was Opti-MEM I Reduced-Serum Medium, Liquid (INVITROGEN catalog number: 31985-070) supplemented with 8% FBS (BIOWEST, catalog number: S1820-500), 200 mg/mL CaCl₂-2H₂O (SIGMA catalog number: C7902-500G), 0.08% chondroitin sulfate (SIGMA catalog number: C9819-5G), 20 µg/mL ascorbic acid (SIGMA catalog number: A4544-25G), 50 µg/mL gentamicin (INVITROGEN catalog number: 15710-064) and 5 ng/mL EGF (INVITROGEN catalog number: PHG 0311) was used as a basal medium. Also, the basal medium supplemented with SB431542 (1 µmol/l) and SB203580 (4-(4-fluorophenyl)-2-(4-methylsulfonylphenyl)-5(4-pyridyl)imidazole <4-[4-(4-fluorophenyl)-2-(4-methylsulfinylphenyl)-1H-imidazol-5-yl]pyridine) (1 µmol/L) (herein also referred to as "SB203580 + SB431542 + 3T3 conditioned medium") was used to culture the corneal endothelial cells. The thus-cultured corneal endothelial cells were amplified by PCR for an SV40 large T antigen and a hTERT gene and introduced into a lentiviral vector (pLenti6.3_V5-TOPO; Life Technologies Inc). Then, 293T cells (RCB2202; Riken Bioresource Center, Ibaraki, Japan) were infected with the lentiviral vector along with three types of helper plasmids (pLP1, pLP2, and pLP/VSVG; Life Technologies Inc.) using a transfection reagent (Fugene HD; Promega Corp., Madison, WI). After 48 hours of infection, a culture supernatant containing the virus was collected and added to a culture solution of the corneal endothelial cells derived from the healthy donor with 5 µg/mL of polybrene to introduce the SV40 large T antigen and the hTERT gene thereinto. Thus, the corneal endothelial cells were immortalized and iHCECs were obtained. The iHCECs were maintained by culturing in DMEM + 10% FBS.

### (Material)

· Immortalized human corneal endothelial cells (iHCEC)
· 150mm dish (Corning 430599)
· Dulbecco's Modified Eagle Medium: DMEM (Nacalai tesque 08456-36)
· 0.5 g/L - Trypsin - 0.53 mmol/L - EDTA Solution, with Phenol Red (Nacalai tesque 32778-34)
· Casein, milk-derived (FUJIFILM Wako Pure Chemical Corporation 030-01505)
· 1 x Phosphatase-Buffered Saline (-): 1 x PBS (-) (Nissui pharma 05913)
· 50mL Centrifuge tube (Corning 430829)
· 0.5% Trypan blue stain (Nakarai tesque 29853-34)

### (Method)

1. Add casein to DMEM to produce cell cleaning solutions with various concentrations of casein.
2. Use immortalized human corneal endothelial cells. Remove a medium from a culture dish in culture, add 1 x PBS (-) warmed to 37°C in advance, and wash. Repeat these procedures twice. Cleaning in this step is intended to remove a culture solution while the cells are still attached to a bottom surface of a culture vessel. The reason why is that a residual protein component such as fetal bovine serum in the culture solution weakens a cell detachment effect of Trypsin in the next step. No loss of the cells occurs because the cells remain in culture and are firmly attached to a basal surface of the culture vessel. The same applies to the following Examples.
3. Remove 1 x PBS (-), and then add 0.05% Trypsin - EDTA and incubate at 37°C (5% CO₂) for 3 minutes.
4. Three minutes later, suspend in 10 mL of a cell cleaning solution and collect in a 50 mL centrifuge tube. A cell concentration in the suspension varies depending on an experiment, but was in a range from 0.5 x 10⁷ to 1 x 10⁷ cells/mL.
5. Count the number of viable cells by trypan blue staining.
6. Centrifuge at 300G for 5 minutes.
7. Remove a supernatant and suspend in 10 mL of a cell cleaning solution.
8. Centrifuge at 300G for 5 minutes.
9. Repein Steps 7 and 8 again.
10. Remove a supernatant, suspend in DMEM, and count the number of viable cells by trypan blue staining again.
11. Calculate a loss rate of the cells from the number of viable cells counted in Step 10, taking the number of viable cells counted in Step 5 as 100%.

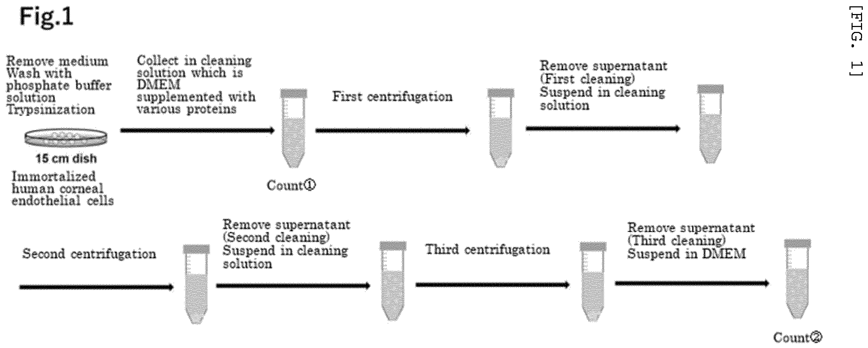

An overview of Example 1 is shown in Figure 1.

### (Results)

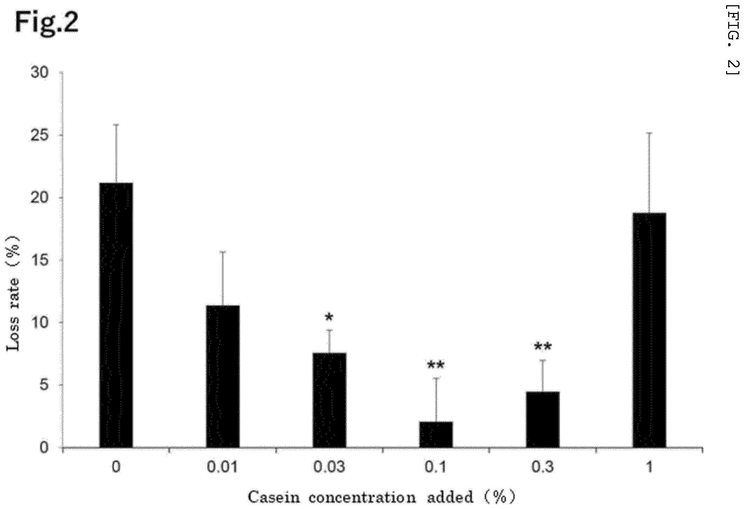

Figure 2 shows a graph of a loss rate when fetal bovine serum was cleaned with cell cleaning solutions including different concentrations of casein. In a conventional cleaning procedure with a 0% group without casein, approximately 20% of the cells were lost during cleaning and 78.8% of the cells were recovered.

A recovery rate of the cells after cleaning was significantly improved in 0.03%, 0.1%, and 0.3% addition groups compared to the 0% group without casein. In particular, when 0.1% casein was added, 97.9% of the cells, that is, almost all cells could be recovered.

### (Example 2: Reduction of loss rate by adding lactoferrin to cell cleaning solution)

### (Material)

· Immortalized human corneal endothelial cells (iHCEC)
· 150mm dish (Corning 430599)
· Dulbecco's Modified Eagle Medium: DMEM (Nacalai tesque 08456-36)
· 0.5 g/L - Trypsin - 0.53 mmol/L - EDTA Solution, with Phenol Red (Nacalai tesque 32778-34)
· Lactoferrin, milk-derived (FUJIFILM Wako Pure Chemical Corporation 127-04122) · 1 x Phosphatase-Buffered Saline (-): 1 x PBS (-) (Nissui pharma 05913)
· 50mL Centrifuge tube (Corning 430829)
· 0.5% Trypan blue stain (Nakarai tesque 29853-34)

### (Method)

1. Add lactoferrin to DMEM to produce cell cleaning solutions with various concentrations of lactoferrin.
2. Use immortalized human corneal endothelial cells. Remove a medium from a culture dish in culture, add 1 x PBS (-) warmed to 37°C in advance, and wash. Repeat these procedures twice.
3. Remove 1 x PBS (-), and then add 0.05% Trypsin - EDTA and incubate at 37°C (5% CO₂) for 3 minutes.
4. Three minutes later, suspend in 10 mL of a cell cleaning solution and collect in a 50 mL centrifuge tube. A cell concentration in the suspension varies depending on an experiment, but was in a range from 0.5 x 10⁷ to 1 x 10⁷ cells/mL.
5. Count the number of viable cells by trypan blue staining.
6. Centrifuge at 300G for 5 minutes.
7. Remove a supernatant and suspend in 10 mL of a cell cleaning solution.
8. Centrifuge at 300G for 5 minutes.
9. Repein Steps 7 and 8 again.
10. Remove a supernatant, suspend in DMEM, and count the number of viable cells by trypan blue staining again.
11. Calculate a loss rate from the number of viable cells counted in Step 10, taking the number of viable cells counted in Step 5 as 100%.

An overview of Example 2 is shown in Figure 1.

### (Results)

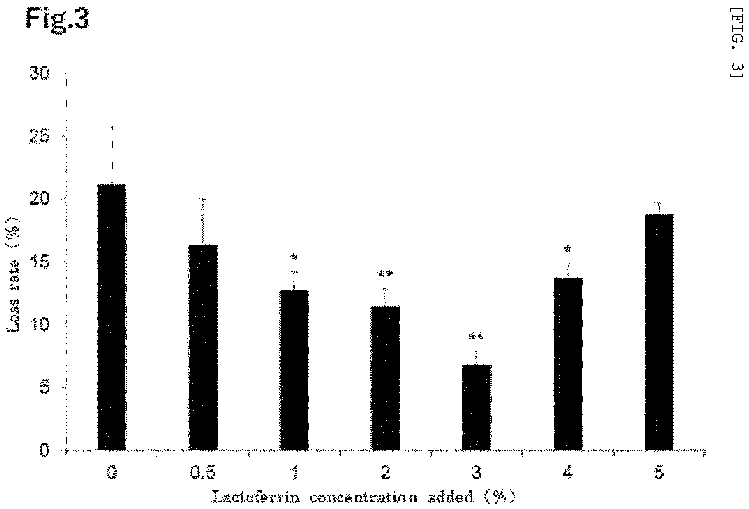

Figure 3 shows a graph of a loss rate when fetal bovine serum was cleaned with cell cleaning solutions including different concentrations of lactoferrin. It was confirmed that a recovery rate was improved in 1%, 2%, 3%, and 4% addition groups compared to a 0% group without lactoferrin.

### (Example 3: Reduction of loss rate by adding ovalbumin to cell cleaning solution)

### (Material)

· Immortalized human corneal endothelial cells (iHCEC)
· 150mm dish (Corning 430599)
· Dulbecco's Modified Eagle Medium: DMEM (Nacalai tesque 08456-36)
· 0.5 g/L - Trypsin - 0.53 mmol/L - EDTA Solution, with Phenol Red (Nacalai tesque 32778-34)
· Albumin, egg-derived (FUJIFILM Wako Pure Chemical Corporation 018-09882)
· 1 x Phosphatase-Buffered Saline (-): 1 x PBS (-) (Nissui pharma 05913)
· 50mL Centrifuge tube (Corning 430829)
· 0.5% Trypan blue stain (Nakarai tesque 29853-34)

### (Method)

1. Add ovalbumin to 1 x PBS (-) to produce a 1.5% solution.
2. Add the solution produced in Step 1 to DMEM to produce cell cleaning solutions with various concentrations of ovalbumin.
3. Use immortalized human corneal endothelial cells. Remove a medium from a culture dish in culture, add 1 x PBS (-) warmed to 37°C in advance, and wash. Repeat these procedures twice.
4. Remove 1 x PBS (-), and then add 0.05% Trypsin - EDTA and incubate at 37°C (5% CO₂) for 3 minutes.
5. Three minutes later, suspend in 10 mL of a cell cleaning solution and collect in a 50 mL centrifuge tube. A cell concentration in the suspension varies depending on an experiment, but was in a range from 0.5 x 10⁷ to 1 x 10⁷ cells/mL.
6. Count the number of viable cells by trypan blue staining.
7. Centrifuge at 300G for 5 minutes.
8. Remove a supernatant and suspend in 10 mL of a cell cleaning solution.
9. Centrifuge at 300G for 5 minutes.
10. Repein Steps 8 and 9 again.
11. Remove a supernatant, suspend in DMEM, and count the number of viable cells by trypan blue staining again.
12. Calculate a loss rate from the number of viable cells counted in Step 11, taking the number of viable cells counted in Step 6 as 100%.

An overview of Example 3 is shown in Figure 1.

### (Results)

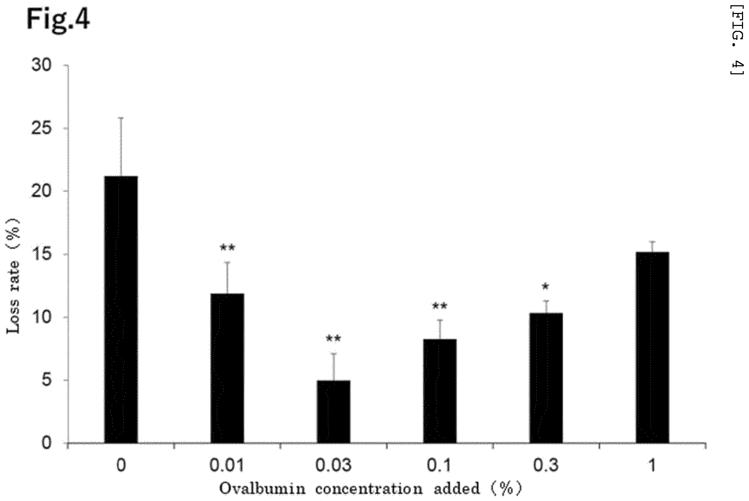

Figure 4 shows a graph of a loss rate when fetal bovine serum was cleaned with cell cleaning solutions including different concentrations of ovalbumin. It was confirmed that a recovery rate was improved in 0.01%, 0.03%, 0.1%, and 0.3% addition groups compared to a 0% group without ovalbumin.

### (Example 4: Reduction of loss rate by adding human serum albumin to cell cleaning solution)

### (Material)

· Immortalized human corneal endothelial cells (iHCEC)
· 150mm dish (Corning 430599)
· OptiMEM^{™}-I (Invitrogen 21585-070)
· Tryple^{™} Select Enzyme (10x) (Thermo Fisher Scientific A12177-01)
· Red Cross Albumin 25% for Intravenous Injection 12.5 g / 50 mL (Japan Blood Products Organization)
· 1 x Phosphatase-Buffered Saline (-): 1 x PBS (-) (Nissui pharma 05913)
· 50mL Centrifuge tube (Corning 430829)
0· .5% Trypan blue stain (Nakarai tesque 29853-34)

### (Method)

1. Add human serum albumin to OptiMEM to produce cell cleaning solutions with various concentrations of human serum albumin.
2. Use immortalized human corneal endothelial cells. Remove a medium from a culture dish in culture, add OptiMEM warmed to 37°C in advance, and wash. Repeat these procedures twice.
3. Remove OptiMEM, and then add Tryple^{™} Select Enzyme (10x) and incubate at 37°C (5% CO₂) for 20 minutes.
4. Twenty minutes later, suspend in 10 mL of a cell cleaning solution and collect in a 50 mL centrifuge tube. A cell concentration in the suspension varies depending on an experiment, but was in a range from 0.5 x 10⁷ to 1 x 10⁷ cells/mL.
5. Count the number of viable cells by trypan blue staining.
6. Centrifuge at 300G for 5 minutes.
7. Remove a supernatant and suspend in 10 mL of a cell cleaning solution.
8. Centrifuge at 300G for 5 minutes.
9. Repein Steps 7 and 8 again.
10. Remove a supernatant, suspend in DMEM, and count the number of viable cells by trypan blue staining again.
11. Calculate a loss rate from the number of viable cells counted in Step 10, taking the number of viable cells counted in Step 5 as 100%.

An overview of Example 4 is shown in Figure 1.

### (Results)

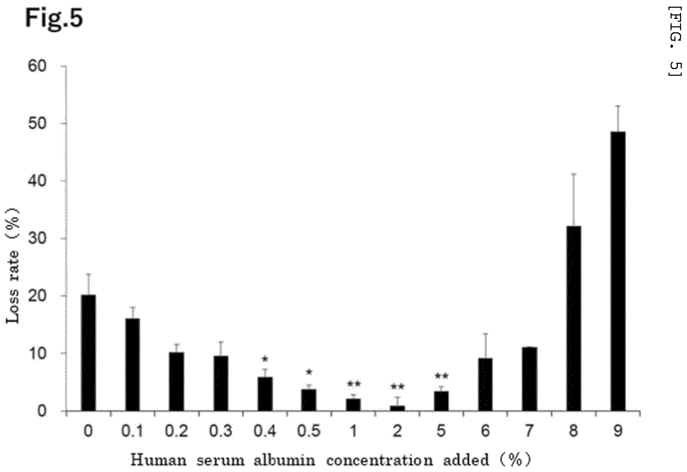

Figure 5 shows a graph of a cell recovery rate when fetal bovine serum was cleaned with cell cleaning solutions including different concentrations of human serum albumin. It was confirmed that the recovery rate was improved in 0.4%, 0.5%, 1%, 2%, and 5% addition groups compared to a 0% group without human serum albumin.

### (Example 5: Reduction of loss rate of non-immortalized corneal endothelial cells for clinical use by adding human serum albumin to cell cleaning solution)

### (Method for culturing human corneal endothelial cells)

Corneal endothelial cells were taken from a human corneal tissue and primary-cultured and passage-cultured. First, a 0.4 w/v% Trypan blue solution for cell staining (FUJIFILM Wako Pure Chemical Corporation, 207-17081) was added to and stained the center of the cornea of a donor. After staining, OptiMEM^{™}-I (invitrogen, 31985-088) to which gentamicin (invitrogen, 15710-064) had been added so as to give a final concentration of 50 µg/mL was used to wash Trypan blue staining solution off. The cornea was observed using a microscope and a monitor installed in a clean bench, and the Descemet's membrane stained by trypan blue was detached from the cornea with forceps. The thus-detached Descemet's membrane was transferred to a 12-well plate containing collagenase (Amano Enzyme Inc. 639-44651) and incubated at 37°C (5% CO2) for 16 hours. After incubation, the Descemet's membrane was transferred to a 15 mL centrifuge tube and centrifuged at 300 G for 3 minutes. After centrifugation, a supernatant was removed, OptiMEM to which gentamicin had been added so as to give a final concentration of 50 µg/mL was added thereto and centrifuged again at 300 G for 3 minutes. These procedures were repeated twice. After centrifugation, a supernatant was removed and a pellet was resuspended in a medium to which Y-27632 (WAKO, 259-00613) had been added so as to give a final concentration of 10 µm. The thus-resuspended cell suspension was seeded into a 6-well plate coated with Laminin E8 fragment (iMatrix-511; Nippi, Incorporated, 892012). After 24 hours, the medium was replaced with a medium without Y-27632, and then culturing was performed for 10 days with the medium being changed every 2 days. OptiMEM^{™}-I to which 8% fetal bovine serum (FBS) (Thermo Fisher Scientific, GVJ0081), 50 µg/mL gentamicin, 200 mg/L calcium chloride (Otsuka Pharmaceutical Co., Ltd. 873215), 0.08% Chondroitin Sulfate C Sodium Salt (WAKO, 032-14613), 5 ng/mL an epidermal growth factor (EGF; invitrogen, PHG0311), 20 µg/mL ascorbic acid (nipro 21F01), and 10 µM SB203580 (Cayman Chemical, 1 3067) had been added was used as a medium.

### (Material)

· Human corneal endothelial cells after 4 passages
· T25 flask (Corning 430168)
· OptiMEM^{™}-! (Invitrogen 21585-070)
· Tryple^{™} Select Enzyme (10x) (Thermo Fisher Scientific A12177-01)
· Red Cross Albumin 25% for Intravenous Injection 12.5 g / 50 mL (Japan Blood Products Organization)
15mL STEMFULL centrifuge tube (Sumitomo Bakelite MS-90150)
0.5% Trypan blue stain (Nakarai tesque 29853-34)

### (Method)

1. Add human serum albumin to OptiMEM to produce cell cleaning solutions with various concentrations of human serum albumin.
2. Use human corneal endothelial cells after 4 passages. Remove a medium from a T25 flask in culture, OptiMEM^{™}-I warmed to 37°C in advance, and wash. Repeat these procedures twice.
3. Remove OptiMEM, and then add Tryple^{™} Select Enzyme (10x) and incubate at 37°C (5% CO₂) for 20 minutes.
4. Twenty minutes later, suspend in 10 mL of a cell cleaning solution and collect in a 15 mL STEMFULL. A cell concentration in the suspension was about 9 x 10⁶ cells/mL.
5. Count the number of viable cells by trypan blue staining.
6. Centrifuge at 300G for 5 minutes.
7. Remove a supernatant and suspend in 10 mL of a cell cleaning solution. A cell concentration in the suspension varies depending on an experiment, but was in a range from 0.5 x 10⁷ to 1 × 10⁷ cells/mL.
8. Centrifuge at 300G for 5 minutes.
9. Repein Steps 7 and 8 again.
10. Remove a supernatant, suspend in OptiMEM, and count the number of viable cells by trypan blue staining again.
11. Calculate a loss rate from the number of viable cells counted in Step 10, taking the number of viable cells counted in Step 5 as 100%.

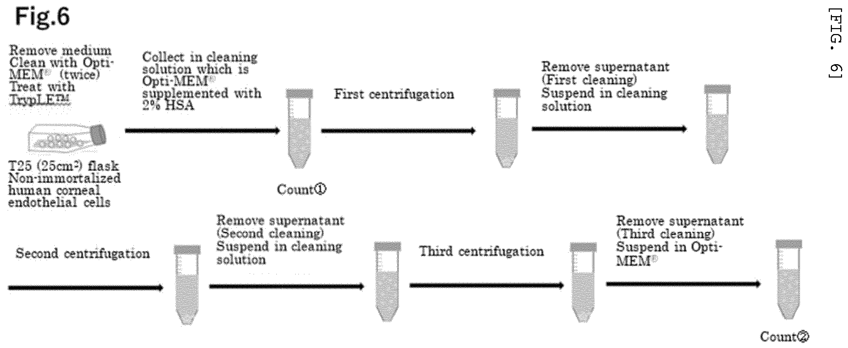

An overview of Example 5 is shown in Figure 6.

### (Results)

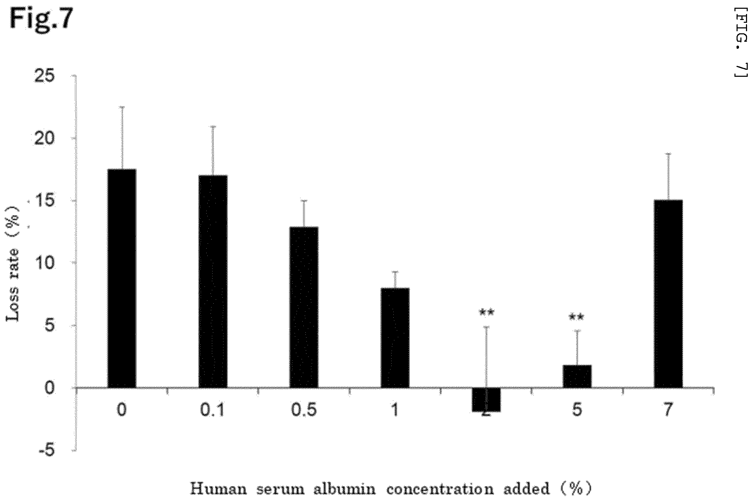

Figure 7 shows a graph of a cell loss rate when fetal bovine serum was cleaned with cell cleaning solutions including different concentrations of human serum albumin. In a conventional cleaning procedure with a 0% group without human serum albumin, approximately 20% of the cells were lost during cleaning and 82.5% of the cells were recovered. It was confirmed that the recovery rate was improved in 2% and 5% addition groups compared to the 0% group without human serum albumin. In particular, addition of 2% human serum albumin to the cell cleaning solution allowed almost all cells to be recovered without loss.

### (Example 6-1: Production Example 1 of corneal endothelial cell formulation)

This example describes an example of production of a corneal endothelial cell formulation (cryopreservation formulation).

### (Material)

| | |
|---|---|
| 2% human serum albumin / CTS-OPTI-MEM | |
| 25% human serum albumin | 3.6mL |
| CTS-OPTI-MEM | 41.4 mL |
| Total | 45 mL |
| 2 x 45mL / 50mL tubes | |

□ Sterile Vial
□ Y27632

### (Method)

· Collect culture supernatant of a corneal endothelial cell culture.
· Suspend corneal endothelial cells in 2% human serum albumin / CTS-OPTI-MEM (30 mL).
· Centrifuge at 300 x g for 5 minutes.
· Suspend in 2% human serum albumin / CTS-OPTI-MEM (20 mL).
· Centrifuge at 300 x g for 5 minutes.
· Suspend in 2% human serum albumin / CTS-OPTI-MEM (20 mL).
· Centrifuge at 300 x g for 5 minutes.
· Suspend Y27632 in CryoStor CS2 dissolved to a concentration of 100 µM and dilute to 2.6 x 10⁶ cells/mL.
· Dispense the above-mentioned cell suspension in 450 µL portions into vials for formulation, and cool the vials to -80°C at a rate of -0.5°C/min using a programmed freezer to freeze the cells.
· Store in a -80°C freezer or under liquid nitrogen.

### (Example 6-2: Production Example 2 of corneal endothelial cell formulation)

This example describes an example of production of a corneal endothelial cell formulation (cryopreservation formulation).

### (Material)

TrypLE^{™} Select Enzyme (10x) (Thermo Fisher Scientific; A1217701)
2% HSA + CTS^{™} Opti-MEM^{™} I Medium (37°C)
CryoStor CS2 (Charles River; 202102)
75 cm² Corning flask for culturing adherent cells
CryoStor CS2 (Charles River; 202102)
Y27632

### (Method)

· Add 5 mL of TrypLE^{™} Select Enzyme (10x) (warmed to 37°C) to a 75 cm² corning flask for culturing adherent cells and incubate at 37°C in an incubator for 20 minutes.
· Collect the entire cell suspension from the 75 cm² corning flask for culturing adherent cells with an autopipettor and place in a 50 mL centrifuge tube (fluid volume: 15 mL).
· Add 10 mL of 2% HSA + CTS^{™} Opti-MEM^{™} I Medium (37°C) to the 75 cm² corning flask for culturing adherent cells to suspend corneal endothelial cells. Repeat these procedures twice (fluid volume: 35 mL).
· Centrifuge the 50 mL centrifuge tube (300 x g, 5 min, room temperature).
· Add 1000 µL of 2% HSA + CTS^{™} Opti-MEM^{™} I Medium to a pellet with a micropipettor and suspend cells by pipetting. Then, wash with 1000 µL without changing a tip.
· Add 18 mL of 2% HSA + CTS^{™} Opti-MEM^{™} I Mediumm (20 mL total).
· Centrifuge the 50 mL centrifuge tube (300 x g, 5 min, room temperature).
· Add 20 mL of 2% HSA + CTS^{™} Opti-MEM^{™} I Medium.
· Centrifuge the 50 mL centrifuge tube (300 x g, 5 min, room temperature).
· Add approximately 4 mL of CryoStor CS2 depending on the number of cells (350 to 450 x 10⁴ cells/mL) .
· Suspend Y27632 in CryoStor CS2 dissolved to a concentration of 450 µM.
· Dispense the above-mentioned cell suspension in 450 µL portions into vials for formulation, and cool the vials to -80°C at a rate from -0.1°C/min to 2.0°C/min using a programmed freezer to freeze the cells.
· Store in a -80°C freezer.

### (Example 7: Use example of corneal endothelial cell formulation)

This example describes an example of use of a corneal endothelial cell formulation.

### (Material)

· Lepus brachyurus
· Frozen corneal endothelial cell formulation prepared in

### Example 6-2

### (Method)

Rabbit lens removal surgery was performed 7 to 14 days prior to cell transfer.
On the day of cell transfer, rabbit corneal endothelium was removed using a scraper over a diameter of 8 mm.
· After the rabbit anterior chamber fluid was removed by aspirating using a syringe with a 30-gauge needle, a corneal endothelial cell formulation (8.0 x 10⁵ HCEC + 0.3 mL CS2 + 100 µM Y-27632), which had been rapidly thawed in a 37°C water bath in advance, was aspirated using a 27-gauge nanoneedle syringe, and 300 µL of the corneal endothelial cell formulation was transferred into the anterior chamber.
After the cell transfer, the rabbit face (plane of the eye) was immobilized in a downward position for 3 hours to allow corneal endothelial cells to engraft in the corneal endothelium.
Five days after the transfer, the rabbit was euthanized for an immunohistological examination.

### (Results)

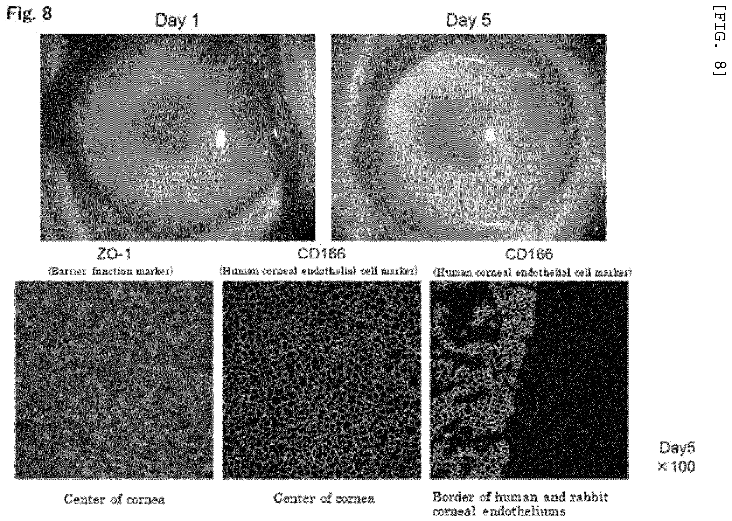

The representative results are shown in Figure 8. It was confirmed that normal corneal endothelial cells were engrafted in the corneal endothelium as indicated by ZO-1, a barrier function marker, and CD166, a human corneal endothelial cell marker. In addition, corneal transparency was restored to normal after 5 days.

Although the present disclosure has been illustrated above with preferred embodiments of the present disclosure, it is to be understood that the present disclosure should be construed only by the claims. It is to be understood that the patents, patent applications, and references cited herein should be incorporated herein by reference in its entirety as if specifically set forth herein. The present application claims the benefit of priority to Japanese Patent Application No. 2022-60148, filed March 31, 2022, the contents of which are incorporated herein by reference.

### [Industrial Applicability]

In the process of formulating corneal endothelial cells cultured for use in regenerative medicine, it is necessary to clean fetal bovine serum derived from a different origin off. Loss upon cell recovery during cell cleaning can be reduced by adding a protein that may be administered to the human body to a cleaning solution. Thus, a cell formulation that has been cleaned with the protein that may be administered to the human body can be used for cell transplantation and the like and is available in the pharmaceutical field.

## Claims

1. A method for producing a corneal endothelial cell formulation, the method comprising:
cleaning corneal endothelial cells or corneal endothelium-like cells with a solution comprising about 0.01% by weight to about 10% by weight of a protein; and
mixing the corneal endothelial cells or the corneal endothelial-like cells with a formulating composition to produce a corneal endothelial cell formulation,
the protein being selected from the group consisting of human serum albumin, casein, lactoferrin, ovalbumin, and a combination thereof.

2. The method according to claim 1, wherein the cleaning comprises suspending the corneal endothelial cells or the corneal endothelium-like cells in the solution to provide a suspension of the corneal endothelial cells or the corneal endothelium-like cells and centrifuging the suspension to recover the corneal endothelial cells or the corneal endothelium-like cells.

3. The method according to claim 1 or 2, wherein the cleaning removes an impurity in the corneal endothelial cell formulation.

4. The method according to any one of claims 1 to 3, wherein a loss rate of the corneal endothelial cells or the corneal endothelial-like cells in the cleaning is 20% or less.

5. The method according to any one of claims 1 to 3, wherein a loss rate of the corneal endothelial cells or the corneal endothelial-like cells in the cleaning is 15% or less.

6. The method according to any one of claims 1 to 3, wherein a loss rate of the corneal endothelial cells or the corneal endothelial-like cells in the cleaning is 10% or less.

7. The method according to any one of claims 1 to 6, wherein the protein is human serum albumin.

8. The method according to claim 7, wherein the solution comprises about 0.5% by weight to about 10% by weight of human serum albumin.

9. The method according to claim 7, wherein the solution comprises about 1% by weight to about 5% by weight of human serum albumin.

10. The method according to claim 7, wherein the solution comprises about 1% by weight to about 3% by weight of human serum albumin.

11. The method according to claim 7, wherein the solution comprises about 2% by weight of human serum albumin.

12. The method according to any one of claims 1 to 11, wherein the solution is a culture medium, a buffer solution, or an intraocular perfusate, or comprises one or more component(s) of the culture medium, the buffer solution, or the intraocular perfusate.

13. The method according to claim 12, wherein the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution, or OPEGUARD.

14. The method according to claim 12, wherein the component of the culture medium comprises calcium, magnesium, potassium, sodium, a phosphate, a bicarbonate, a vitamin, an essential amino acid, or a combination thereof.

15. The method according to claim 14, wherein the component of the culture medium further comprises transferrin, insulin, hypoxanthine, thymidine, sodium bicarbonate, sodium pyruvate, L-glutamic acid, or a combination thereof.

16. The method according to any one of claims 1 to 15, wherein the formulating composition is a cell preservation solution, a culture medium, a buffer solution, or an intraocular perfusate, or comprises one or more component(s) of the cell preservation solution, the culture medium, the buffer solution, or the intraocular perfusate.

17. The method according to claim 16, wherein the cell preservation solution is CryoStor (registered trademark) CS2, CryoStor (registered trademark) CS5, Bambanker (registered trademark), or CellBanker (registered trademark).

18. The method according to claim 16, wherein the component of the cell preservation solution comprises DMSO, glycerin, polyethylene glycol, or a combination thereof.

19. A method for cleaning corneal endothelial cells or corneal endothelium-like cells, the method comprising:
suspending the corneal endothelial cells or the corneal endothelium-like cells in a solution comprising about 0.01% by weight to about 10% by weight of a protein to provide a suspension of the corneal endothelial cells or the corneal endothelium-like cells; and
centrifuging the suspension to recover the corneal endothelial cells or the corneal endothelium-like cells,
the protein being selected from the group consisting of human serum albumin, casein, lactoferrin, ovalbumin, and a combination thereof.

20. A cell cleaning solution for corneal endothelial cells or corneal endothelium-like cells, the solution comprising
about 0.01% by weight to about 10% by weight of a protein selected from the group consisting of human serum albumin, casein, lactoferrin, ovalbumin, and a combination thereof.

21. The cell cleaning solution according to claim 20, wherein cleaning of the corneal endothelial cells is performed by suspending the corneal endothelial cells or the corneal endothelium-like cells in the cell cleaning solution to provide a suspension of the corneal endothelial cells or the corneal endothelium-like cells and centrifuging the suspension to recover the corneal endothelial cells or the corneal endothelium-like cells.

22. The cell cleaning solution according to claim 20 or 21, wherein the cleaning removes an impurity in the corneal endothelial cell formulation.

23. The cell cleaning solution according to any one of claims 20 to 22, wherein a loss rate of the corneal endothelial cells or the corneal endothelial-like cells in the cleaning is 20% or less.

24. The cell cleaning solution according to any one of claims 20 to 23, wherein a loss rate of the corneal endothelial cells or the corneal endothelial-like cells in the cleaning is 15% or less.

25. The cell cleaning solution according to any one of claims 17 to 19, wherein a loss rate of the corneal endothelial cells or the corneal endothelial-like cells in the cleaning is 10% or less.

26. The cell cleaning solution according to any one of claims 17 to 22, wherein the protein is human serum albumin.

27. The cell cleaning solution according to claim 26, wherein the solution comprises about 0.5% by weight to about 10% by weight of human serum albumin.

28. The cell cleaning solution according to claim 26, wherein the solution comprises about 1% by weight to about 5% by weight of human serum albumin.

29. The cell cleaning solution according to claim 26, wherein the solution comprises about 1% by weight to about 3% by weight of human serum albumin.

30. The cell cleaning solution according to claim 26, wherein the solution comprises about 2% by weight of human serum albumin.

31. The cell cleaning solution according to any one of claims 1 to 30, wherein the solution is a culture medium, a buffer solution, or an intraocular perfusate, or comprises one or more component(s) of the culture medium, the buffer solution, or the intraocular perfusate.

32. The cell cleaning solution according to claim 31, wherein the culture medium or the intraocular perfusate is Opti-MEM (registered trademark), DMEM, MEM, RPMI 1640, Ham's F-12, PBS, HEPES, Hanks' Balanced Salt Solution (HBSS), a saline solution, an oxyglutathione solution.

33. The cell cleaning solution according to claim 31, wherein the component of the culture medium comprises calcium, magnesium, potassium, sodium, a phosphate, a bicarbonate, a vitamin, an essential amino acid, or a combination thereof.

34. The cell cleaning solution according to claim 31, wherein the component of the culture medium further comprises transferrin, insulin, hypoxanthine, thymidine, sodium bicarbonate, sodium pyruvate, L-glutamic acid, or a combination thereof.

35. A composition for producing a corneal endothelial cell formulation in a suspension state, the composition comprising
about 0.01% by weight to about 10% by weight of a protein selected from the group consisting of human serum albumin, casein, lactoferrin, ovalbumin, and a combination thereof.
